# EUROPEAN PATENT APPLICATION

(11) **EP 4 803 099 A1**
(43) Date of publication of application: **09.09.2026**
(21) Application number: 24885879.7
(22) Date of filing: 01.11.2024
(51) Int. Cl.: A61K 45/00, A61K 31/47, A61K 31/496, A61K 31/498, A61K 31/517, A61K 31/4375, A61K 31/5025, A61K 31/5377, A61K 31/7088, A61K 38/02, A61K 39/395, A61K 45/06, A61K 48/00, A61P 15/00, A61P 15/14, A61P 35/00, A61P 43/00

(54) **CANCER-ASSOCIATED FIBROBLAST INHIBITOR**

(30) Priority: 02.11.2023 JP 2023188377
(71) Applicant: Teikyo University, Tokyo 173-8605 (JP)
(72) Inventor: OKAMOTO, Koji, Tokyo 173-8605 (JP); MORI, Yutaro, Tokyo 173-8605 (JP)
(74) Representative: Witthoff Jaekel Steinecke Patentanwälte PartG mbB
(86) International application number: PCT/JP2024/039118
(87) International publication number: WO 2025/095115

(57) **Abstract**

Provided is a cancer-associated fibroblast inhibitor, particularly a cancer-associated fibroblast inhibitor that can enhance the anticancer effects of anticancer agents. The cancer-associated fibroblast inhibitor comprises a PDGFR inhibitor.

## Description

### Technical Field

The present invention relates to a cancer-associated fibroblast inhibitor and the like.

### Background Art

Although molecular targeted therapy and immunotherapy have had a major impact on refractory cancers, many tumors eventually acquire chemoresistance. Such resistance is due to intratumor heterogeneity, which allows some cancer cells to survive and grow after treatment. Notably, cancer cells acquire chemoresistance by forming cellular networks with non-tumor cells such as cancer-associated fibroblasts (CAFs) (NPL 1). Therefore, to understand the biological basis of resistance, it is important to grasp the intratumor heterogeneity and the overall picture of intercellular networks that allow resistant cancer cells to survive.

### Citation List

### Non-patent Literature

NPL 1: Nat Rev Clin Oncol 18, 792-804. 10.1038/s41571-021-00546-5.

### Summary of Invention

### Technical Problem

An object of the present invention is to provide a cancer-associated fibroblast inhibitor, particularly a cancer-associated fibroblast inhibitor that can enhance the anticancer effects of anticancer agents.

### Solution to Problem

As a result of extensive research in view of the above problems, the present inventors found that PDGFR inhibitors can suppress cancer-associated fibroblasts and enhance the anticancer effects of anticancer agents. As a result of further research based on this finding, the present inventors have completed the present invention. Specifically, the present invention includes the following embodiments.

Item 1. A cancer-associated fibroblast inhibitor comprising a PDGFR inhibitor.

Item 1A. A method for inhibiting cancer-associated fibroblasts, comprising applying a PDGFR inhibitor to a subject (particularly a subject in need of inhibition of cancer-associated fibroblasts).

Item 1B. A PDGFR inhibitor for use in the inhibition of cancer-associated fibroblasts.

Item 1C. Use of a PDGFR inhibitor for producing a cancer-associated fibroblast inhibitor.

Item 1D. Use of a PDGFR inhibitor for inhibiting cancer-associated fibroblasts.

Item 2. The cancer-associated fibroblast inhibitor according to Item 1, wherein the PDGFR inhibitor is at least one selected from the group consisting of a PDGFR function inhibitor and a PDGFR expression inhibitor.

Item 3. The cancer-associated fibroblast inhibitor according to Item 1 or 2, wherein the PDGFR inhibitor is at least one selected from the group consisting of a low-molecular-weight compound, a polynucleotide targeting PDGFR, an expression cassette for the polynucleotide, a peptide, a protein, and an antibody.

Item 4. The cancer-associated fibroblast inhibitor according to any one of Items 1 to 3, wherein the PDGFR inhibitor is a low-molecular-weight compound, and the low-molecular-weight compound is a kinase inhibitor.

Item 5. The cancer-associated fibroblast inhibitor according to Item 4, wherein the kinase inhibitor is at least one selected from the group consisting of ripretinib, ponatinib, erdafitinib, dovitinib, lenvatinib, foretinib, ENMD-2076, PP121, and cediranib.

Item 6. The cancer-associated fibroblast inhibitor according to any one of Items 1 to 5, wherein the cancer-associated fibroblasts are cells in ovarian cancer tissue, breast cancer tissue, or colon cancer tissue.

Item 7. The cancer-associated fibroblast inhibitor according to any one of Items 1 to 6, wherein the cancer-associated fibroblasts are cells in ovarian cancer tissue.

Item 8. The cancer-associated fibroblast inhibitor according to any one of Items 1 to 7, for use in combined administration with an anticancer agent.

Item 9. The cancer-associated fibroblast inhibitor according to Item 8, wherein the anticancer agent is a platinum-based drug.

Item 10. An enhancer of the anticancer effect of an anticancer agent, the enhancer comprising a PDGFR inhibitor.

Item 10A. A method for enhancing the anticancer effect of an anticancer agent, comprising applying a PDGFR inhibitor to a subject (particularly a subject in need of enhancement of the anticancer effect of an anticancer agent).

Item 10B. A PDGFR inhibitor for use in the enhancement of the anticancer effect of an anticancer agent.

Item 10C. Use of a PDGFR inhibitor for producing an enhancer of the anticancer effect of an anticancer agent.

Item 10D. Use of a PDGFR inhibitor for enhancing the anticancer effect of an anticancer agent.

Item 11. A preventive or therapeutic agent for at least one cancer selected from the group consisting of ovarian cancer, breast cancer, colon cancer, pancreatic cancer, urothelial cancer, prostate cancer, esophageal cancer, liver cancer, kidney cancer, uterine cancer, stomach cancer, glioblastoma, lung cancer, and melanoma, the preventive or therapeutic agent comprising a PDGFR inhibitor.

Item 11A. A method for preventing or treating at least one cancer selected from the group consisting of ovarian cancer, breast cancer, colon cancer, pancreatic cancer, urothelial cancer, prostate cancer, esophageal cancer, liver cancer, kidney cancer, uterine cancer, stomach cancer, glioblastoma, lung cancer, and melanoma, comprising applying a PDGFR inhibitor to a subject (particularly a subject in need of prevention or treatment of at least one cancer selected from the group consisting of ovarian cancer, breast cancer, colon cancer, pancreatic cancer, urothelial cancer, prostate cancer, esophageal cancer, liver cancer, kidney cancer, uterine cancer, stomach cancer, glioblastoma, lung cancer, and melanoma).

Item 11B. A PDGFR inhibitor for use in the prevention or treatment of at least one cancer selected from the group consisting of ovarian cancer, breast cancer, colon cancer, pancreatic cancer, urothelial cancer, prostate cancer, esophageal cancer, liver cancer, kidney cancer, uterine cancer, stomach cancer, glioblastoma, lung cancer, and melanoma.

Item 11C. Use of a PDGFR inhibitor for producing a preventive or therapeutic agent for at least one cancer selected from the group consisting of ovarian cancer, breast cancer, colon cancer, pancreatic cancer, urothelial cancer, prostate cancer, esophageal cancer, liver cancer, kidney cancer, uterine cancer, stomach cancer, glioblastoma, lung cancer, and melanoma.

Item 11D. Use of a PDGFR inhibitor for preventing or treating at least one cancer selected from the group consisting of ovarian cancer, breast cancer, colon cancer, pancreatic cancer, urothelial cancer, prostate cancer, esophageal cancer, liver cancer, kidney cancer, uterine cancer, stomach cancer, glioblastoma, lung cancer, and melanoma.

Item 12. The preventive or therapeutic agent according to Item 11, for use in combined administration with an anticancer agent.

### Advantageous Effects of Invention

According to the present invention, it is possible to provide a cancer-associated fibroblast inhibitor, particularly a cancer-associated fibroblast inhibitor that can enhance the anticancer effects of anticancer agents.

### Brief Description of Drawings

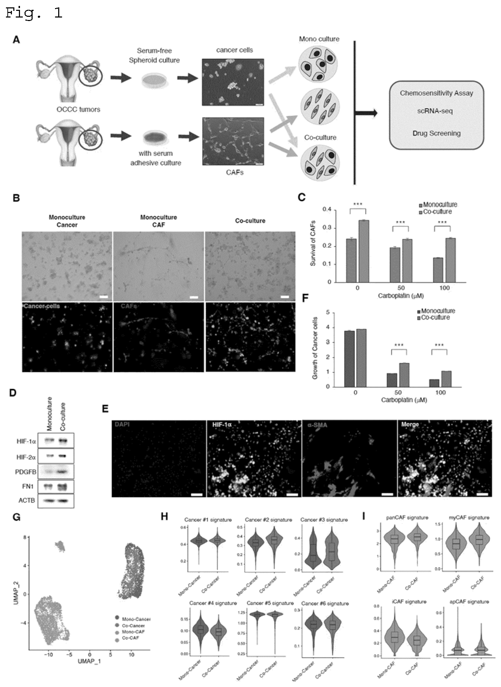

Fig. 1 shows that co-cultivation of CAFs with chemoresistant OCCC cells recapitulates the chemoresistant niche *in vitro*. (A) Experimental design of the *in vitro* co-culture system. Cancer spheroid cells and CAFs were derived from surgical specimens of HIF-1α-positive OCCC. The established cancer cells and CAFs were labeled with GFP/Luc2 and mCherry/hRluc, respectively, cultivated either alone or in combination, and subjected to a chemosensitivity assay, scRNA-seq, or drug screening. (B) Bright-phase images (top) and fluorescence images (bottom) of the indicated cells cultivated under organoid conditions for 7 days. Scale bars: 100 µm. (C) Survival of CAFs upon monoculture or co-culture with cancer cells for 7 days. Cultured cells were grown in the absence or presence of the indicated concentrations of carboplatin, and cell survival was evaluated by measuring hRLuc activity. p values were determined by Student's t test. (D) Western blot analyses of cancer cells that were FACS-sorted after incubation under monoculture or co-culture conditions for 3 days. (E) Representative images of immunostaining of HIF-1α and α-SMA in cancer cells and CAFs co-cultured for 3 days. Scale bars: 100 µm. (F) Cancer cell growth upon monoculture or co-culture with CAFs for 7 days. Cultured cells were grown in the absence or presence of the indicated concentrations of carboplatin, and cancer cell proliferation was evaluated by measuring Luc2 activity. (G) UMAP plot of scRNA-seq data from cancer cells and CAFs incubated under monoculture and co-culture conditions for 3 days. (H) Violin plots of the signature scores for the cancer subpopulations (Cancer #1-6) grown under the monoculture and co-culture conditions in (G). (I) Violin plots of the indicated signature genes in CAFs grown under the monoculture and co-culture conditions shown in (G): ∗∗∗p < 0.001.

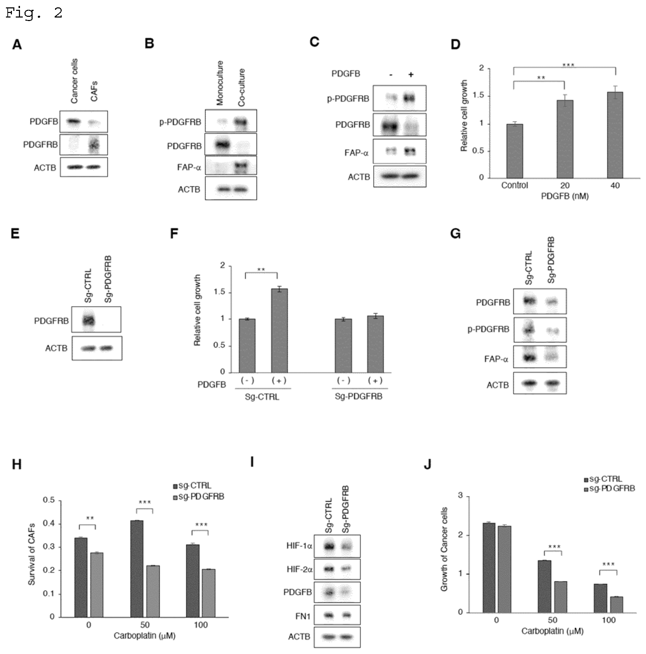

Fig. 2 shows that CAF activation by cancer-derived PDGF mediates chemoresistance and HIF-1α activation of cancer cells. (A) Western blot analysis of GFP-labeled cancer cells and mCherry-labeled CAFs with the indicated antibodies. (B) Western blot analysis of CAFs grown under monoculture or co-culture conditions for 3 days. Note that a PDGFRB level was reduced under co-culture conditions, presumably via negative feedback regulation. (C) Western blot analyses of CAFs treated with 40 nM PDGFB for 3 days. (D) Relative growth of CAFs treated with different concentrations of PDGFB for 7 days. (E) Western blot analyses of CAFs subjected to Cas9/CRIPSR-mediated knockout. (F) Relative growth of CAFs transduced with the indicated sgRNA and then treated with 20 nM of PDGFB for 7 days. (G) Western blot analyses of control and PDGFRB-deficient CAFs that were FACS-sorted on mCherry after incubation with cancer cells for 3 days. (H) Survival of control and PDGFRB-deficient CAFs that were incubated with cancer cells for 7 days. (I) Proliferation of cancer cells cultured for 7 days with control or PDGFRB-deficient CAFs in the presence of the indicated concentrations of carboplatin. (J) Western blot analysis of cancer cells that were FACS-sorted on GFP after incubation for 3 days with control or PDGFRB-deficient CAFs. p values were determined by Student's t test. Statistically significant differences are indicated: ∗∗p < 0.01, ∗∗∗p < 0.001.

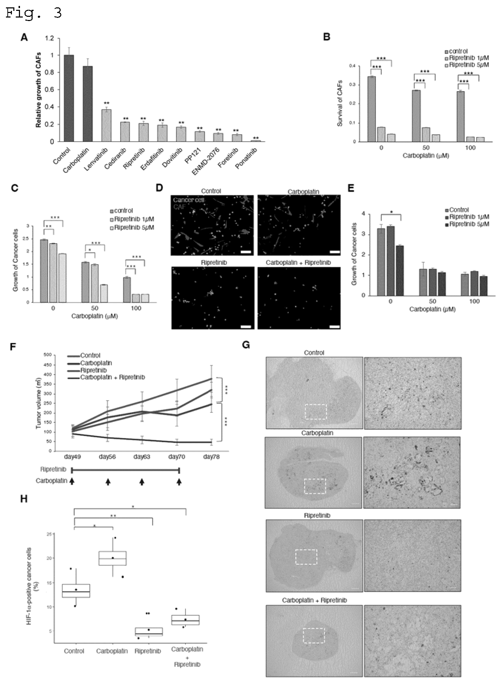

Fig. 3 shows that CAF inhibition by ripretinib blocks growth of OCCC in combination with carboplatin. (A) Inhibition of ovarian cancer-derived CAFs cultured for 7 days in the presence of the indicated TKIs or carboplatin (1 µM). (B) Inhibition of CAFs by ripretinib. CAFs co-cultured with cancer cells were treated with the indicated concentrations of ripretinib and carboplatin for 7 days. (C) Co-operative inhibition of the growth of co-cultivated cancer cells by ripretinib and carboplatin. Cancer cells co-cultured with CAFs were treated for 7 days with the indicated concentrations of ripretinib and carboplatin. (D) Fluorescence images of cancer cells and CAFs co-cultured for 7 days in the presence or absence of 100 µM carboplatin and/or 5 µM ripretinib. Scale bars: 100 µm. (E) Cancer cells grown under monoculture conditions were treated with the indicated concentrations of ripretinib and carboplatin for 7 days. (F) Tumor xenograft mice were treated with the indicated combinations of carboplatin and/or ripretinib, and tumor volume (mean ± standard error of the mean) was measured weekly. The number of days after cancer cell transplantation is indicated. (G) Immunostaining of xenograft tumors (78 days post-transplantation) with HIF-1α. Magnified images are shown in the right panels. Scale bars: 500 µm (right panels) and 100 µm (left panels). (H) Boxplots showing the percentage fraction of HIF-1α-positive cancer cells in the tumor tissues shown in (G). Average values ± SEM are shown. p values were determined by Student's t test. Statistically significant differences are indicated: ∗p < 0.05, ∗∗p < 0.01, ∗∗∗p < 0.001.

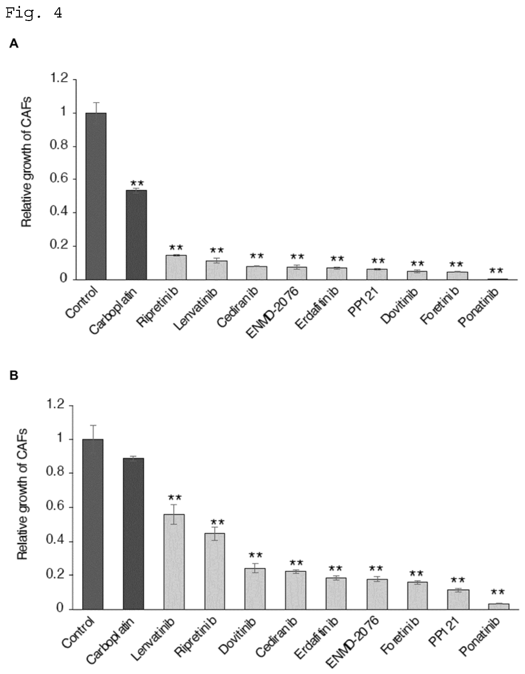

Fig. 4 shows inhibition of breast cancer-derived CAFs (A) and colon cancer-derived CAFs (B) cultured for 7 days in the presence of the indicated TKIs or carboplatin (1 µM). Average values ± SEM are shown. p values were determined by Student's t test. Statistically significant differences are indicated: ∗p < 0.05, ∗∗p < 0.01, ∗∗∗p < 0.001.

### Description of Embodiments

The terms "containing" and "comprising" as used herein include the concepts of "containing," "comprising," "consisting essentially of," and "consisting of."

In one embodiment, the present invention relates to a cancer-associated fibroblast inhibitor comprising a PDGFR inhibitor, an enhancer of the anticancer effect of an anticancer agent, and a preventive or therapeutic agent for ovarian cancer and/or breast cancer (which are also collectively referred to herein as "the agent of the present invention"). This is described below.

### (1) Active ingredient

### (1-1) Inhibition target

The PDGFR gene encodes the platelet-derived growth factor receptor, which is a tyrosine kinase. PDGFR (PDGFR protein or PDGFR mRNA) to be inhibited is an expression product of the PDGFR gene, and is PDGFR protein or PDGFR mRNA expressed by an organism or its cells (particularly cancer-associated fibroblasts) to which the agent of the present invention is applied. Therefore, the PDGFR protein and PDGFR mRNA to be inhibited can also be changed as necessary, depending on the biological species of the subject. Examples of the biological species include, but are not particularly limited to, animals such as various mammals, including humans, monkeys, mice, rats, dogs, cats, rabbits, pigs, horses, cows, sheep, goats, and deer.

Examples of PDGFR include PDGFRα and PDGFRβ. The amino acid sequences of PDGFR proteins and the base sequences of PDGFR mRNAs derived from various biological species are known. Specifically, for example, the human PDGFRα gene is identified by NCBI gene ID 5156, and the human PDGFRβ gene is identified by NCBI gene ID 5159. The amino acid sequences and base sequences in various biological species can be obtained or estimated from this information. PDGFR proteins and PDGFR mRNAs may also include splicing variants of those described above.

The PDGFR protein to be inhibited may have amino acid mutations, such as substitutions, deletions, additions, and insertions, as long as it retains its original properties, i.e., PDGF-binding properties and tyrosine kinase activity. From the viewpoint that activity is less likely to be impaired, mutations are preferably substitutions, and more preferably conservative substitutions.

The PDGFR mRNA to be inhibited may also have base mutations, such as substitutions, deletions, additions, and insertions, as long as the protein translated from the mRNA has its original properties, i.e., PDGF-binding properties and tyrosine kinase activity. Preferred mutations are those that do not result in amino acid substitutions in the protein translated from the mRNA or those that result in conservative amino acid substitutions.

Preferred specific examples of the PDGFR protein to be inhibited include proteins consisting of an amino acid sequence having 85 to 100% identity with the amino acid sequence of a wild-type PDGFR protein, and having PDGF-binding properties and tyrosine kinase activity. The identity is more preferably 90% or more, even more preferably 95% or more, and still even more preferably 98% or more.

Preferred specific examples of the PDGFR mRNA to be inhibited include those consisting of a base sequence that has 85 to 100% identity with the base sequence of wild-type PDGFR mRNA, and that encodes proteins having PDGF-binding properties and tyrosine kinase activity. The identity is more preferably 90% or more, even more preferably 95% or more, and still even more preferably 98% or more.

The "identity" of amino acid sequences refers to the degree to which two or more comparable amino acid sequences match each other. Therefore, the more identical two amino acid sequences are, the higher the identity or similarity of those sequences. The level of amino acid sequence identity is determined, for example, using FASTA, a sequence analysis tool, with default parameters. Alternatively, it can be determined using the BLAST algorithm by Karlin and Altschul (Karlin S, Altschul SF. "Methods for assessing the statistical significance of molecular sequence features by using general scoring schemes," Proc Natl Acad Sci USA. 87: 2264-2268 (1990); Karlin S, Altschul SF. "Applications and statistics for multiple high-scoring segments in molecular sequences," Proc Natl Acad Sci USA. 90: 5873-7 (1993)). A program called "BLASTX," based on this BLAST algorithm, has been developed. The specific techniques of these analysis methods are known and can be found on the website of the National Center of Biotechnology Information (NCBI) (http://www.ncbi.nlm.nih.gov/). The "identity" of base sequences is also defined in the same manner as above.

The term "conservative substitutions" as used herein means substitutions of amino acid residues with other amino acid residues having similar side chains. For example, substitutions between amino acid residues having basic side chains, such as lysine, arginine, and histidine, correspond to conservative substitutions. In addition, the following substitutions between amino acid residues also correspond to conservative substitutions: substitution between amino acid residues having acidic side chains, such as aspartic acid and glutamic acid; substitution between amino acid residues having uncharged polar side chains, such as glycine, asparagine, glutamine, serine, threonine, tyrosine, and cysteine; substitution between amino acid residues having nonpolar side chains, such as alanine, valine, leucine, isoleucine, proline, phenylalanine, methionine, and tryptophan; substitution between amino acid residues having β-branched side chains, such as threonine, valine, and isoleucine; and substitution between amino acid residues having aromatic side chains, such as tyrosine, phenylalanine, tryptophan, and histidine.

### (1-2) Inhibitor

The PDGFR inhibitor is not particularly limited as long as it is a component that can inhibit the function and/or expression of PDGFR. The PDGFR inhibitor is preferably a low-molecular-weight compound, a polynucleotide targeting PDGFR, an expression cassette for the polynucleotide, a peptide, a protein, an antibody, or the like. The PDGFR inhibitors can be used singly or in combination of two or more.

### (1-2-1) PDGFR function inhibitor

The PDGFR function inhibitor is not particularly limited as long as it can inhibit the function of PDGFR protein and/or mRNA expressed in an organism or its cells (particularly cancer-associated fibroblasts) to which the agent of the present invention is applied. PDGFR function inhibitors can be used singly or in combination of two or more.

The PDGFR function inhibitor is not particularly limited as long as it can reduce tyrosine kinase activity and/or inhibit ligand (PDGF) binding. Specific examples include tyrosine kinase inhibitors and antagonists.

PDGFR function inhibitors include not only those that act specifically on PDGFR, but also those that are specific to tyrosine kinases other than PDGFR (e.g., KIT, ABL, VEGFR, SRC, FGFR, FLT, and other PDGFR-related tyrosine kinases) but also act on PDGFR. In addition, PDGFR function inhibitors also include those that act on multiple tyrosine kinases including PDGFR (e.g., the PDGFR-related tyrosine kinases mentioned above).

Examples of PDGFR function inhibitors include low-molecular-weight compounds (for example, with a molecular weight of 1000 or less, 800 or less, 700 or less, or 600 or less, and, for example, with a molecular weight of 100 or more, 150 or more, or 200 or more).

Various low-molecular-weight compounds that can inhibit PDGFR function are commercially available, and many have been reported so far in a variety of literature. Examples of tyrosine kinase inhibitors, which are such low-molecular-weight compounds, include ripretinib, ponatinib, erdafitinib, dovitinib, lenvatinib, foretinib, ENMD-2076, PP121, and cediranib. Among these, ripretinib is particularly preferred.

Examples of other PDGFR function inhibitors include PDGFR antibodies. PDGFR antibodies are preferably antibodies that bind to the PDGF-binding region of PDGFR. The binding site can be determined based on known information and/or can be predicted based on known information (e.g., by docking model construction).

Antibodies include polyclonal antibodies, monoclonal antibodies, chimeric antibodies, single-chain antibodies, and portions of these antibodies that have antigen-binding properties, such as Fab fragments or fragments produced by Fab expression libraries. The antibodies of the present invention also include antibodies that have antigen-binding properties to polypeptides typically consisting of at least 8 amino acids, preferably 15 amino acids, and more preferably 20 amino acids, that are consecutive in the amino acid sequence of PDGFR. These antibodies are commercially available. For example, known anti-PDGFR antibodies include ab67017 produced by Abcam, STJ117738 produced by St John's Laboratory, and LS-C766558-60 produced by LifeSpan BioSciences.

In addition to the above, any molecules that can bind (preferably specifically bind) to PDGFR (e.g., peptides, proteins, artificial antibodies, and aptamers) can be used as PDGFR function inhibitors. When a protein (e.g., an antibody) or a peptide is used as a PDGFR function inhibitor, an expression cassette thereof can also be used instead.

### (1-2-2) PDGFR expression inhibitor

The PDGFR expression inhibitor is not particularly limited as long as it can inhibit the expression level of PDGFR protein and/or PDGFR mRNA in an organism or its cells (particularly cancer-associated fibroblasts) to which the agent of the present invention is applied. PDGFR expression inhibitors can be used singly or in combination of two or more.

Examples of PDGFR expression inhibitors include PDGFR-specific small interfering RNA (siRNA), PDGFR-specific microRNA (miRNA), PDGFR-specific antisense nucleic acids, and expression cassettes thereof; PDGFR-specific ribozymes; and PDGFR gene editing agents using the CRISPR/Cas system.

"Inhibition of expression" means inhibiting the expression levels of PDGFR protein, PDGFR mRNA, etc. to, for example, 1/2, 1/3, 1/5, 1/10, 1/20, 1/30, 1/50, 1/100, 1/200, 1/300, 1/500, 1/1000, or 1/10,000 or less, and also includes reducing their expression levels to 0.

### (1-2-2-1) siRNA, miRNA, and antisense nucleic acids

The PDGFR-specific siRNA is not particularly limited as long as it is a double-stranded RNA molecule that specifically inhibits the expression of the gene encoding PDGFR. In an embodiment, the siRNA preferably has a length of, for example, 18 bases or more, 19 bases or more, 20 bases or more, or 21 bases or more. Further, the siRNA preferably has a length of, for example, 25 bases or less, 24 bases or less, 23 bases or less, or 22 bases or less. The upper and lower limits of the length of siRNA described here are assumed to be combinable in any manner.

The structure of the siRNA is not particularly limited. The siRNA may be shRNA (small hairpin RNA). The siRNA may have additional bases at the 5'- or 3'-end. The siRNA may have a protruding sequence (overhang) at the 3'-end, and specific examples include those with dTdT (dT stands for deoxythymidine) added.

The sequences of the siRNA and/or shRNA can be searched for, for example, using search software provided free of charge on various websites. Examples of such websites include the following: siRNA Target Finder (http://www.ambion.com/jp/techlib/misc/siRNA_finder.html) and pSilencer^{TM} Expression Vectors Insert Design Tool (http://www.ambion.com/jp/techlib/misc/psilencer_converter.html) provided by Ambion, and GeneSeer (http://codex.cshl.edu/scripts/newsearchhairpin.cgi) provided by RNAi Codex.

The PDGFR-specific miRNA may be any miRNA as long as it inhibits the translation of the gene encoding PDGFR. For example, instead of cleaving the target mRNA like siRNA, the miRNA may bind to the 3'-untranslated region (UTR) of the target and inhibit its translation. The miRNA may be any of pri-miRNA (primary miRNA), pre-miRNA (precursor miRNA), and mature miRNA. The length of the miRNA is not particularly limited. The length of pri-miRNA is generally several hundred to several thousand bases, the length of pre-miRNA is generally 50 to 80 bases, and the length of mature miRNA is generally 18 to 30 bases. In an embodiment, the PDGFR-specific miRNA is preferably pre-miRNA or mature miRNA, and more preferably mature miRNA. The PDGFR-specific miRNA may be synthesized by a known method or purchased from a company that provides synthetic RNA.

The PDGFR-specific antisense nucleic acid is a nucleic acid that contains a base sequence complementary or substantially complementary to the base sequence of mRNA of the gene encoding PDGFR, or a portion thereof, and that has the function of inhibiting PDGFR protein synthesis by binding to the mRNA and forming a specific and stable double strand. The antisense nucleic acid may be any of DNA, RNA, and DNA/RNA chimera. When the antisense nucleic acid is DNA, the RNA:DNA hybrid formed by the target RNA and antisense DNA is recognized by endogenous ribonuclease H (RNase H), leading to selective degradation of the target RNA. Therefore, in the case of antisense DNA that directs degradation by RNase H, the target sequence may be not only a sequence in mRNA, but also a sequence in an intron region in the initial translation product of the PDGFR gene. The intron sequence can be determined by comparing the genomic sequence with the cDNA base sequence of the PDGFR gene using a homology search program such as BLAST or FASTA. The length of the target region of the PDGFR-specific antisense nucleic acid is not limited as long as hybridization of the antisense nucleic acid to the target region results in the inhibition of translation into PDGFR protein. The PDGFR-specific antisense nucleic acid may be the entire or partial sequence of the mRNA encoding PDGFR. Considering ease of synthesis, antigenicity, intracellular internalization issues, and the like, oligonucleotides consisting of about 10 to about 40 bases, particularly about 15 to about 30 bases, are preferred, but are not limited thereto. More specifically, the 5'-end hairpin loop, 5'-end untranslated region, translation initiation codon, protein coding region, ORF translation termination codon, 3'-end untranslated region, 3'-end palindrome region, 3'-end hairpin loop, or the like of the PDGFR gene may be selected as a preferred target region for the antisense nucleic acid, but is not limited thereto.

The PDGFR-specific siRNA, PDGFR-specific miRNA, PDGFR-specific antisense nucleic acid, and the like can be prepared by determining the target sequence of mRNA or an initial transcription product based on the cDNA sequence or genomic DNA sequence of the PDGFR gene, and synthesizing a sequence complementary thereto using a commercially available automated DNA/RNA synthesizer. In addition, antisense nucleic acids containing various modifications can also be chemically synthesized by known methods.

The expression cassette for the PDGFR-specific siRNA, PDGFR-specific miRNA, or PDGFR-specific antisense nucleic acid is not particularly limited as long as it is a polynucleotide into which the PDGFR-specific siRNA, PDGFR-specific miRNA, or PDGFR-specific antisense nucleic acid has been incorporated in an expressible state. Typically, the expression cassette contains a promoter sequence and a polynucleotide containing a coding sequence for the PDGFR-specific siRNA, PDGFR-specific miRNA, or PDGFR-specific antisense nucleic acid (and optionally a transcription termination signal sequence), and optionally other sequences.

The terms "nucleic acid" and "polynucleotide" as used herein are not particularly limited and include both natural and artificial ones. Specifically, in addition to DNA, RNA, and the like, those with known chemical modifications may be used, as exemplified below. To prevent degradation by hydrolases such as nucleases, the phosphate residue of each nucleotide can be substituted with a chemically modified phosphate residue such as phosphorothioate (PS), methylphosphonate, or phosphorodithioate. In addition, the hydroxyl group at the 2-position of the sugar (ribose) of each ribonucleotide may be replaced with -OR (R represents, for example, CH3(2'-O-Me), CH2CH2OCH3(2'-O-MOE), CH2CH2NHC(NH)NH2, CH2CONHCH3, or CH2CH2CN). Furthermore, the base moiety (pyrimidine, purine) may be chemically modified, for example, by introducing a methyl group or a cationic functional group into the 5-position of the pyrimidine base, or by replacing the carbonyl group at the 2-position with a thiocarbonyl group. Further examples include, but are not limited to, those in which the phosphate moiety or hydroxyl moiety is modified with, for example, biotin, an amino group, a lower alkylamine group, or an acetyl group. In addition, BNA (LNA), which is a nucleotide in which the 2' oxygen and 4' carbon of the sugar moiety are bridged to fix the conformation of the sugar moiety in the N-type, can also be used.

### (1-2-2-2) Gene editing agent

The PDGFR gene editing agent is not particularly limited as long as it can inhibit the expression of the PDGFR gene using a target sequence-specific nuclease system (e.g., the CRISPR/Cas system). The expression of the PDGFR gene can be inhibited, for example, by disrupting the PDGFR gene or by modifying the promoter of the PDGFR gene to inhibit the activity of the promoter.

For example, when the CRISPR/Cas system is used, the PDGFR gene editing agent for use can typically be, but is not limited to, a vector containing a guide RNA expression cassette targeting the PDGFR gene or its promoter, and a Cas protein expression cassette (PDGFR gene editing vector). In addition to this typical example, for example, a combination of a vector containing a guide RNA targeting the PDGFR gene or its promoter and/or its expression cassette, and a vector containing a Cas protein and/or its expression cassette can also be used as the PDGFR gene editing agent.

The guide RNA is not particularly limited as long as it is used in the CRISPR/Cas system. For example, various ones can be used that can bind to a target site in genomic DNA (e.g., the PDGFR gene or its promoter) and also bind to a Cas protein, thereby guiding the Cas protein to the target site in genomic DNA.

The "target site" as used herein is a site on genomic DNA that consists of a DNA strand (target strand) consisting of a PAM (proto-spacer adjacent motif) sequence and a sequence of approximately 17 to 30 bases long (preferably 18 to 25 bases long, more preferably 19 to 22 bases long, and particularly preferably 20 bases long) adjacent to the 5' side of the PAM sequence, and its complementary DNA strand (non-target strand).

The guide RNA has a sequence involved in binding to the target site in genomic DNA (also referred to as the "crRNA (CRISPR RNA) sequence"), and this crRNA sequence binds complementarily (preferably complementarily and specifically) to a sequence excluding the sequence complementary to the PAM sequence in the non-target strand, thereby enabling the guide RNA to bind to the target site in genomic DNA. The guide RNA also has a sequence involved in binding to the Cas protein (also referred to as the "tracrRNA (trans-activating crRNA) sequence"), and by binding to the Cas protein, this tracrRNA sequence can guide the Cas protein to the target site in genomic DNA.

The tracrRNA sequence is not particularly limited. The tracrRNA sequence is typically an RNA sequence of approximately 50 to 100 bases long that can form multiple (generally three) stem-loops, and the sequence varies depending on the type of Cas protein used. Any of various known sequences can be used as the tracrRNA sequence, depending on the type of Cas protein used.

The guide RNA generally contains the crRNA and tracrRNA sequences described above. The guide RNA may be a single-stranded RNA (sgRNA) containing a crRNA sequence and a tracrRNA sequence, or an RNA complex formed by complementary binding of an RNA containing a crRNA sequence and an RNA containing a tracrRNA sequence.

The Cas protein is not particularly limited as long as it can be used in the CRISPR/Cas system. For example, various Cas proteins can be used that can bind to a target site in genomic DNA in a complex with a guide RNA and cleave the target site. Cas proteins are known to be derived from various organisms, such as Cas9 protein, and more preferably Cas9 protein endogenously contained in bacteria belonging to the genus *Streptococcus*. Information on the amino acid sequences of various Cas proteins and their coding sequences can be easily obtained from various databases such as NCBI.

PDGFR gene editing agents can be easily produced according to known genetic engineering techniques. For example, they can be produced using PCR, restriction enzyme digestion, DNA ligation techniques, *in vitro* transcription/translation techniques, recombinant protein production techniques, and the like.

### (2) Use

As clarified in the Examples provided below, the PDGFR expression inhibitor has an inhibitory effect on cancer-associated fibroblasts (inhibitory effects on proliferation and activation). Therefore, the PDGFR expression inhibitor can be used as an active ingredient in cancer-associated fibroblast inhibitors.

The cancer tissue in which the target cancer-associated fibroblasts are present is not particularly limited. Examples include ovarian cancer, breast cancer, uterine cancer, colon cancer, pancreatic cancer, urothelial cancer, prostate cancer, esophageal cancer, liver cancer, kidney cancer, uterine cancer, stomach cancer, glioblastoma, lung cancer, melanoma, leukemia, lung cancer, and skin cancer. Preferred among these are ovarian cancer, breast cancer, colon cancer, pancreatic cancer, urothelial cancer, prostate cancer, esophageal cancer, liver cancer, kidney cancer, uterine cancer, stomach cancer, glioblastoma, lung cancer, and melanoma; more preferred are ovarian cancer, breast cancer, and colon cancer; even more preferred are ovarian cancer and breast cancer; and particularly preferred is ovarian cancer.

Ovarian cancer is not particularly limited. Examples include superficial, epithelial, and stromal malignant tumors (e.g., serous (cystic) adenocarcinoma, mucinous (cystic) adenocarcinoma, endometrioid adenocarcinoma, clear cell adenocarcinoma, adenocarcinoma-fibroma (all types listed above), adenosarcoma, mesodermal mixed tumor (Müllerian mixed tumor or carcinosarcoma), malignant Brenner tumor, transitional cell carcinoma, and undifferentiated carcinoma), sex cord-stromal tumors (e.g., fibrosarcoma and Sertoli-stromal cell tumor (poorly differentiated)), germ cell tumors (e.g., dysgerminoma, yolk sac tumor (endodermal sinus tumor), embryonal cancer (fetal cancer), polyembryoma, choriocarcinoma, mature cystic teratoma with malignant transformation, and immature teratoma (G3)), carcinoma, sarcoma, malignant lymphoma (primary), and secondary (metastatic) tumors. Among these, adenocarcinoma is particularly preferred, with clear cell carcinoma being particularly preferred.

The type of cancer targeted is desirably a chemoresistant cancer. Although the definition of chemoresistant cancer varies depending on the type of cancer, in an embodiment, the chemoresistant cancer can be defined as a case in which no therapeutic effect (no tumor shrinkage) is observed with first-line anticancer agent treatment (e.g., a platinum-based drug in the case of ovarian cancer), or a case in which although a therapeutic effect (tumor shrinkage) is temporarily observed, early regrowth occurs thereafter. In addition, the proportion of HIF-1α-positive cells among cancer cells in cancer tissue can be used as an indicator, and if this proportion is 10% or higher, the cancer can be determined to be chemoresistant.

The cancer-associated fibroblasts are particularly preferably myofibroblastic cancer-associated fibroblasts, from the viewpoint of the inhibitory effect on chemoresistance in cancer. The cancer-associated fibroblasts can be characterized by the expression or high expression of αSMA and/or collagen I. Further, the myofibroblastic cancer-associated fibroblasts mentioned above can be characterized by the expression or high expression of FAP, TPM1, THBS2 (particularly FAP-α), and the like. In an embodiment, the target cancer may be a cancer that contains cancer-associated fibroblasts/myofibroblastic cancer-associated fibroblasts characterized by the expression of the above markers.

Since chemoresistance is caused by cancer-associated fibroblasts, inhibiting cancer-associated fibroblasts with the PDGFR expression inhibitor can enhance the anticancer effects of anticancer agents against chemoresistant cancer. From this point of view, the PDGFR expression inhibitor can be used as an active ingredient in enhancers of the anticancer effects of anticancer agents. Furthermore, since chemoresistance is acquired due to cancer-associated fibroblasts, it is possible to suppress the acquisition of chemoresistance in cancer by inhibiting cancer-associated fibroblasts with the PDGFR expression inhibitor. For these reasons, the PDGFR expression inhibitor is suitable for combined administration with an anticancer agent.

The active ingredient of the present invention can be used, for example, as a medicament, a reagent, a food composition, an oral composition, a health enhancer, or a nutritional product (e.g., a supplement), and further, together with an anticancer agent, as a composition for improving cancer (e.g., a medicament, a reagent, a food composition, an oral composition, a health enhancer, or a nutritional product (e.g., a supplement)). The active ingredient of the present invention can be used as is, or combined with conventional ingredients to form various compositions and applied (e.g., administered, ingested, inoculated, or treated) to animals, humans, and various cells.

The target for application is not particularly limited. Examples of target mammals include humans, monkeys, mice, rats, dogs, cats, rabbits, pigs, horses, cows, sheep, goats, and deer.

When the active ingredient of the present invention is administered in combination with an anticancer agent, the combination includes not only cases in which they are applied simultaneously, but also cases in which they are applied at intervals (for example, at intervals of several minutes to several days (e.g., 1 minute to 10 days)).

When the active ingredient of the present invention is used as a preventive or therapeutic agent for cancer, the preventive or therapeutic agent may be one for use in combined administration with an anticancer agent, for example. In this case, the preventive or therapeutic agent may contain an anticancer agent. Further in this case, the active ingredient of the present invention and the anticancer agent may be contained in the same container, or the active ingredient of the present invention and the anticancer agent may be contained in separate containers.

Examples of anticancer agents include platinum-based drugs, metabolic antagonists, alkylating agents, microtubule inhibitors, antibiotic anticancer agents, topoisomerase inhibitors, molecular targeted drugs, hormone agents, and biological drugs, and particularly preferably platinum-based drugs.

Examples of platinum-based drugs include cisplatin, carboplatin, nedaplatin, oxaliplatin, satraplatin, miriplatin, lobaplatin, spiroplatin, tetraplatin, ormaplatin, and iproplatin.

Examples of metabolic antagonists include enocitabine, carmofur, capecitabine, tegafur, tegafur-uracil, tegafur-gimeracil-oteracil potassium, gemcitabine, cytarabine, cytarabine ocfosfate, nelarabine, fluorouracil, fludarabine, pemetrexed, pentostatin, methotrexate, cladribine, doxifluridine, hydroxycarbamide, and mercaptopurine.

Examples of alkylating agents include cyclophosphamide, ifosfamide, nitrosourea, dacarbazine, temozolomide, nimustine, busulfan, melphalan, procarbazine, and ranimustine.

Examples of microtubule inhibitors include alkaloid anticancer agents such as vincristine; and taxane anticancer agents such as docetaxel and paclitaxel.

Examples of antibiotic anticancer agents include mitomycin C, doxorubicin, epirubicin, daunorubicin, bleomycin, actinomycin D, aclarubicin, idarubicin, pirarubicin, peplomycin, mitoxantrone, amrubicin, and zinostatin stimalamer.

Examples of topoisomerase inhibitors include CPT-11, irinotecan, and nogitecan, which have topoisomerase I inhibitory action; and etoposide and sobuzoxane, which have topoisomerase II inhibitory action.

Examples of hormone agents include dexamethasone, finasteride, tamoxifen, astrozole, exemestane, ethinylestradiol, chlormadinone, goserelin, bicalutamide, flutamide, prednisolone, leuprorelin, letrozole, estramustine, toremifene, fosfestrol, mitotane, methyltestosterone, medroxyprogesterone, and mepitiostane.

Examples of biological drugs include interferon α, interferon β, interferon γ, interleukin 2, ubenimex, and dry BCG.

The agent of the present invention may further contain one or more other components as necessary. These other components are not particularly limited, as long as they can be incorporated into medicaments, food compositions, oral compositions, health enhancers, nutritional products (e.g., supplements), and the like. Examples include bases, carriers, solvents, dispersants, emulsifiers, buffers, stabilizers, excipients, binders, disintegrants, lubricants, thickeners, humectants, colorants, fragrances, and chelating agents. Examples of pharmaceutically acceptable carriers and additives include, but are not limited to, excipients such as sucrose and starch; binders such as cellulose and methylcellulose; disintegrants such as starch and carboxymethylcellulose; lubricants such as magnesium stearate and Aerosil; flavorings such as citric acid and menthol; preservatives such as sodium benzoate and sodium bisulfite; stabilizers such as citric acid and sodium citrate; suspending agents such as methylcellulose and polyvinylpyrroliden; dispersants such as surfactants; diluents such as water and physiological saline; and base waxes. The form of the agent of the present invention is not particularly limited, and can take a form generally used in each use according to the purpose of use.

When the agent of the present invention is used as a medicament, it can be in any dosage form. Examples of dosage forms include oral dosage forms, such as tablets (including orally disintegrating tablets, chewable tablets, effervescent tablets, lozenges, and jelly-like drops), pills, granules, fine granules, powders, hard capsules, soft capsules, dry syrups, liquids (including health drinks, suspensions, and syrups), and jelly formulations; and parenteral dosage forms, such as injectable formulations (e.g., drip infusions (e.g., formulations for intravenous drip infusion), intravenous injections, intramuscular injections, subcutaneous injections, and intradermal injections), topical agents (e.g., ointments, plasters, and lotions), suppositories, inhalants, ophthalmic formulations, ophthalmic ointments, nasal drops, ear drops, and liposome formulations.

The administration route of the agent of the present invention is not particularly limited, as long as the desired effect can be obtained. Examples include oral administration; and parenteral administration including enteral administration, such as tube-feeding and enema administration, intravenous administration, intraarterial administration, intramuscular administration, intracardiac administration, subcutaneous administration, intradermal administration, and intraperitoneal administration.

Examples of the form for use in health enhancers, nutritional products (e.g., supplements), and the like include dosage forms suitable for oral administration (oral dosage forms), such as tablets (including orally disintegrating tablets, chewable tablets, effervescent tablets, lozenges, and jelly-like drops), pills, granules, fine granules, powders, hard capsules, soft capsules, dry syrups, liquids (including health drinks, suspensions, and syrups), and jelly formulations.

Examples of the form for use in food compositions include liquid, gel, or solid foods, such as juices, soft drinks, teas, soups, soy milk, salad oils, dressings, yogurt, jellies, puddings, sprinkles, milk powder for childcare, cake mixes, powder or liquid dairy products, bread, and cookies.

The content of the active ingredient of the present invention in the agent of the present invention varies depending on, for example, the type of active ingredient, the application, the mode of use, the target of application, and the condition of the target, and is not limited. For example, the content of the active ingredient is 0.0001 to 100 wt.%, and preferably 0.001 to 50 wt.%.

The amount of application (e.g., administration, ingestion, or inoculation) of the agent of the present invention is not particularly limited as long as it is an effective amount that exhibits the desired effect, and is generally 0.01 to 1000 mg/kg of body weight per day in terms of the weight of the active ingredient. The above dosage can be administered once a day or in divided doses (2 or 3 times) per day, and can also be increased or decreased as appropriate depending on the age, disease state, and symptoms.

### Examples

The present invention is described in detail below based on Examples; however, the present invention is not limited by the Examples.

### 1. Test method

### 1-1. Nucleus isolation

Frozen ovarian clear cell carcinoma (OCCC) samples were homogenized in 500 µl of ice-cold Nuclei EZ Lysis buffer (NUC-101, Sigma-Aldrich) using a KIMBLE Dounce tissue grinder (D8938, Sigma-Aldrich), and incubated on ice for 5 minutes with an additional 1 ml of the lysis buffer. The homogenate was filtered through a 70-µm cell strainer (#352350, Corning) and then centrifuged at 500×g for 1 minute at 4°C. The pellet was resuspended, washed with 1 ml of lysis buffer, and incubated on ice for 5 minutes. After another cycle of washing with lysis buffer, the pellet was washed twice in 1 ml of Nuclei Suspension Buffer (1x PBS, 1% BSA, 0.2% RNase inhibitor (2313A, Clontech/TaKaRa)). The nuclear pellet was resuspended in 1 ml of Nuclei Suspension Buffer and filtered twice through a 35-µm cell strainer (#352235, Corning).

### 1-2. Single-nucleus RNA-seq (snRNA-seq)

For snRNA-seq of OCCC tissues, cDNA libraries were prepared from isolated nuclei (4000-8000 nuclei) on a Chromium controller (10X Genomics) using the Single Cell 3' Reagent Kit v3 (PN-1000075, 10X Genomics). Next-generation sequencing of the cDNA libraries was performed on the HiSeq 2500 (Illumina) platform at a median depth of 65,124 reads/cell. Fastq files of the sequencing data were processed by the cellranger pipeline (version 3.0.2, 10X Genomics) and mapped to the GRCh38 (version 3.0.0 for premRNA) reference genome to generate matrices of unique molecular identifiers (UMIs) and cell-associated barcodes.

### 1-3. Spatial transcriptomics

Frozen OCCC samples were embedded in pre-chilled OCT compound (#25608-930, Sakura Finetek Japan Co., Ltd.), re-frozen on dry ice, and then stored at -80°C. Preparation of cDNA libraries from the tissue sections was performed using a Visium Spatial Gene Expression kit (10X Genomics) according to the manufacturer's instructions. Optimal parameters for permeabilization of OCCC tissue were determined using the Visium Spatial Tissue Optimization Kit (PN-1000193; 10x Genomics). Subsequently, 10 mm sections cut from OCT-embedded samples were subjected to H&E staining and cDNA library preparation from barcoded Visium spots after permeabilization for 20 minutes. Next-generation sequencing was performed on a HiSeq 2500 (Illumina) platform. Fastq files of the sequencing data were processed by the spaceranger pipeline (version 1.1.0., 10X Genomics) and mapped to the GRCh38 reference genome to generate matrices of UMI and spot-associated barcodes.

### 1-4. Targeted genome sequencing

Genomic DNA was extracted from frozen OCCC tissues using a DNeasy Blood & Tissue kit (#69504, Qiagen) prior to selection of targeted sequences using the SureSelect NCC Oncopanel (v.4.0; Agilent Technologies). Subsequently, libraries were constructed using a SureSelectXT reagent kit (Agilent Technologies). Paired-end sequencing (2 × 150 bp) was performed by using NextSeq 500 (Illumina). Mutations (single-nucleotide variations, short insertions, and deletions), gene amplifications, and gene fusions were detected using the cisCall system.

### 1-5. Bulk RNA-seq analysis

Total RNA extraction and library preparation were performed as previously reported. Briefly, total RNA was extracted from frozen samples of OCCC (30 cases) using TRIzol (#15596026, Invitrogen), and then subjected to cDNA library preparation by using a TruSeq Stranded mRNA Library Prep Kit (RS-20020595, Illumina) according to the manufacturer's instructions. Subsequently, cDNA libraries were sequenced on the Illumina HiSeq 2500 platform with the 2 × 100-bp paired-end read module. Sequenced reads were mapped onto the human genome reference sequence (UCSU hg19) using Basespace (Illumina).

### 1-6. Establishment of tumor-derived spheroids and cancer-associated fibroblasts (CAFs)

OCCC tissues obtained by surgical excision were washed immediately with PBS, cut into pieces 10 mm³ or less using a scalpel, and dissociated with collagenase/hyaluronidase (#7912, Stem Cell Technologies) for 2 hours at 37°C. Dissociated cells were filtered sequentially through 100- and 70-µm cell strainers (352350, BD Falcon) and isolated by density gradient centrifugal purification in PBS containing Histodenz (D2158, Sigma). After lysis of red blood cells with ACK Lysing Buffer (A1049201, Thermo Fisher Scientific), the isolated cells were used to establish cancer spheroids by cultivating them on ultra-low-attachment culture dishes (#3471 or #3262, Corning) in STEMPRO hESC SFM (A1000701, Thermo Fisher Scientific) supplemented with 8 ng/mL basic fibroblast growth factor (#AA10-155, Thermo Fisher Scientific) and penicillin/streptomycin (37°C, 5% CO₂). Serial passage of formed cancer spheroids was performed every 2 weeks by dissociating the spheroids with Accumax (AM105, Innovative Cell Technologies). To establish CAF cultures, the red blood cell-removed Histodenz-purified cells were grown on attachment culture dishes (#35003, Corning) in MEM-α (#12561-05, Thermo Fisher Scientific) containing 10% FBS (#10270106, Thermo Fisher Scientific) and penicillin/streptomycin (37°C, 5% CO₂). For serial passage of established CAFs, attached cells were dissociated with TripLE Express Enzyme (#12604013, Thermo Fisher Scientific) every 2 weeks.

In addition, CAFs were established from breast cancer tissue and colon cancer tissue in the same manner as described above. CAFs derived from breast cancer tissue and CAFs derived from colon cancer tissue were used only in the test shown in Fig. 4.

### 1-7. Plasmid construction

To generate the pCDH-Luc2-T2A-copGFP plasmid, the Luc2-T2A-copGFP cassette was initially generated by ligating a synthesized T2A sequence to Luc2 (PCR-amplified from pGL4.51[Luc2/CMV/Neo] (Promega, E1320)) and copGFP (PCR-amplified from pCDH-CMV-MCS-EF1α-copGFP (System Biosciences, CD511B-1)). Subsequently, the Luc2-T2A-TagBFP sequence in pCDH-Luc2-T2A-TagBFP was substituted with the Luc2-T2A-copGFP cassette via the EcoRI and SalI sites to generate pCDH-Luc2-T2A-copGFP. To generate the pCDH-hRluc-T2A-mCherry plasmid, the hRluc-T2A-mCherry cassette was first generated by ligating the synthesized T2A sequence with hRluc (PCR-amplified from pGL4.74[hRluc/TK] (Promega, E6921)) and mCherry (PCR-amplified from pcDNA5-MTS-TagBFP-P2AT2A-EGFP-NLS-P2AT2A-mCherry-PTS1 (Addgene, #87829)). Subsequently, pCDH-hRluc-T2A-mCherry was generated from pCDH-Luc2-T2A-TagBFP using a similar construction strategy. The pCDH-Luc2-T2A-copGFP and pCDH-hRluc-T2A-mCherry plasmids were used to generate lentiviruses for gene transduction into cancer cells and CAFs, respectively.

### 1-8. In vitro co-culture assay

OCCC spheroid cells and CAFs were infected by lentiviruses expressing Luc2 and GFP (pCDH-Luc2-T2A-copGFP), and lentiviruses expressing hRLuc and mCherry (pCDH-hRLuc-T2A-mCherry), respectively. For co-culture, the infected spheroid cells and CAFs were mixed at a 1:1 ratio. Subsequently, monocultured cancer cells, monocultured CAFs, or co-cultured cells were plated into a 96-well plate (1 × 10⁴ cells/well) layered with growth factor-reduced (GFR) Matrigel (#356231, Corning), and then incubated for 6 hours with MEM-α supplemented with 10% FBS. After removing floating dead cells, the remaining cells were overlaid with GFR Matrigel and subsequently with E medium (DMEM/F12-GlutaMAX (#10565-042, Thermo Fisher Scientific)) supplemented with penicillin-streptomycin, 10 mM HEPES (#15630106, Thermo Fisher Scientific), N-2 Supplement (#17502-001, Thermo Fisher Scientific), B-27 supplement (#17504-001, Thermo Fisher Scientific), 1 mM N-acetylcysteine (A7250, Sigma-Aldrich), and 50 ng/mL human EGF (PHG0313, Thermo Fisher Scientific). For the chemosensitivity assay, monocultured or co-cultured cells were treated with carboplatin (S1215, Selleck Chemicals). Cell growth was evaluated using a dual luciferase reporter kit (E1960, Promega). For western blot analysis, cultivated cells were harvested with Cell Recovery Solution (#354253, Corning), and GFP-expressing cancer cells and mCherry-expressing CAFs were selected by flow cytometry (FACS Aria III, Beckton Dickinson, Franklin Lakes, New Jersey).

### 1-9. Single-cell RNA-seq (scRNA-seq) of in vitro-cultivated cells

Monoculture and co-culture of cancer cells and CAFs (3 days after incubation) were used to prepare single-cell cDNA libraries. For this, 3000-6000 cells were applied to a Chromium controller (10X Genomics). Library construction was performed using the Single Cell 3' Reagent Kit v3 and 3' CellPlex Kit Set A (10X Genomics) according to the manufacturer's instructions. Next-generation sequencing of the cDNA libraries was performed using a HiSeq 2500 (Illumina). Fastq files of the sequencing data were processed by the cellranger pipeline (version 6.1.2, 10X Genomics) using the "cellranger multi" command, and then mapped onto the GRCh38 reference genome to generate matrices of UMI and cell-associated barcodes.

### 1-10. In vitro proliferation assays of CAFs

To examine the chemosensitivity of CAFs, *in vitro-*cultivated CAFs at Day 7-10 post-passage were enzymatically dissociated and used for chemosensitivity assays. The following tyrosine kinase inhibitors (TKIs) were purchased from Selleck Chemicals and used for the assays: carboplatin (S1215), lenvatinib (S1164), cediranib (S1017), ripretinib (S8757), erdafitinib (S8401), dovitinib (S1018), PP121 (S2622), ENMD-2076 (S1181), foretinib (S1111), and ponatinib (S1490). Human recombinant PDGFB (160-24033, Fuji Film Wako) was used to examine the effects of PDGF signaling on CAFs. The effects of TKIs or PDGFB on cell growth were quantified by measuring luciferase activity in a CellTiter-Glo Luminescent Cell Viability Assay (G7571, Promega) according to the manufacturer's instructions.

### 1-11. CRISPR/Cas9-mediated gene knockout

CAFs grown for 7-10 days after passaging were enzymatically dissociated and subjected to Cas9-mediated gene knockout using the Neon Transfection System (Thermo Fisher Scientific) according to the manufacturer's instructions. The sgRNA/Cas9 complex formed after mixing Cas9 protein (Invitrogen) with Edit-R Human Synthetic sgRNA pool for PDGFRB (SQ-003163-01-0002, Dharmacon-Horizon Discovery) or Edit-R Synthetic sgRNA Non-targeting Control #1 (U-009501-01-001p, Dharmacon-Horizon Discovery) was used for electroporation (1600 V, 10 ms, two pulses).

### 1-12. Animal experiments

To examine the synergistic effects of carboplatin and ripretinib on xenograft tumors, Luc2-GFP-labeled cancer spheroid cells and HRluc-mCherry-labeled CAFs were dissociated and mixed at a 1:1 ratio. Next, 1 × 10⁵ mixed cells were suspended in 100 µL of E medium containing 50% GFR Matrigel, and injected subcutaneously into the flanks of NOG (NOD/Shi-scid IL-2Rγnull) mice (Japan Crea). At 49 days post-transplantation (tumor volume: 100 mm³ or less), mice were randomized into four groups and with/without carboplatin (40 mg/kg per week, intraperitoneal injection) and/or ripretinib (50 mg/kg/day, oral administration) for an additional 28 days. Tumor volume was calculated weekly using the standard formula: length × width × height × π/6. Evaluation of tumor volume according to luciferase activity was performed using the IVIS Spectrum imaging system (Caliper Life Science). This system measured total luminescence emitted from the area of the mouse abdomen (photons/sec/cm2/sr) at 10 min post-intraperitoneal administration of 15 mg/mL D-luciferin potassium salt (10 µL per g body weight, Wako). The data were analyzed using Living Image software (v. 4.2; Caliper Life Science).

### 1-13. Western blot analyses

Western blot analyses were performed as previously described. Antibodies specific for the following markers were purchased from the indicated suppliers: PAX8 (10336-1-AP, Proteintech; dilution 1:2000), Cytokeratin 7 (M7018, Dako; 1:1000), α-SMA (ab7817, Abcam; 1:3000), Collagen I (ab138492, Abcam; 1:1000), β-Actin (A5316, Sigma-Aldrich; 1:1000), HIF-1α (ab51608, Abcam; 1:1000), HIF-2α (ab199, Abcam; 1:1000), PDGFB (ab23914, Abcam; 1:1000), Fibronectin (ab268020, Abcam; 1:1000), PDGFRB (#3169, Cell Signaling Technology; 1:1000), PDGFRB (phospho Y1021; ab16868, Abcam; 1:1000), and FAP-α (ab53066, Abcam; 1:1000).

### 1-14. Immunofluorescence analysis of clinical specimens

To immunostain clinical samples of OCCC, surgical specimens were fixed in 10% formaldehyde, embedded in paraffin, and sliced into 4-mm sections. For histological examination, sections were stained with H&E. For immunofluorescence analyses, sections were subjected to antigen retrieval with 10 mM citric acid buffer (pH: 6.0), followed by blocking of endogenous peroxidase activity with 0.3% hydrogen peroxide. For co-staining with PAX8, HIF-1α, and α-SMA, slides were stained sequentially with a rabbit anti-PAX8 antibody (1:1000; Proteintech, 10336-1-AP), biotinylated-goat anti-rabbit IgG (1:500; Vector Laboratories, BA-1000), the Vectastain Elite ABC detection kit (Vector Laboratories, PK-6100), and Alexa Fluor^{TM} 488 Tyramide Reagent (Invitrogen, B40953). For consecutive staining with anti-HIF-1α and anti-α-SMA antibodies, slides were boiled in 10 mM citric acid buffer (pH: 6.0) for 15 minutes or more to remove the PAX8-secondary antibody complex. Slides were then stained with a rabbit anti-HIF-1α antibody (1:100; Abcam, ab51608) and a mouse anti-α-SMA antibody (1:600; Abcam, ab7817), followed by donkey anti-rabbit IgG AlexaFluor 750-conjugated (1:1000; Abcam, ab175728) or goat anti-mouse IgG-Alexa Fluor 555-conjugated (1:1000; Invitrogen, A21424) secondary antibodies. The immunostained slides were then mounted using ProLong^{™} Diamond Antifade Mountant containing DAPI (Invitrogen, P36971). The same procedure was used for immunostaining with anti-KRT7, anti-α-SMA, and anti-PDGFRB (phospho Y1021) antibodies. Antibodies specific for the following markers were purchased from the listed suppliers: Cytokeratin 7 (M7018, Dako, 1:100), α-SMA (ab7817, Abcam, 1:600), and PDGFRB (phospho Y1021) (ab16868, Abcam, 1:100). Immunofluorescence images were evaluated by Vectra Polaris (Akoya Biosciences).

### 1-15. Immunofluorescence analysis of in vitro-cultivated cells

Cancer cells under monoculture conditions or under co-culture conditions with CAFs were plated on GFR Matrigel-coated glass-bottom dishes (D11140H, Matsunami Glass Ind., Ltd.), fixed with cold methanol, and permeabilized with 0.1% Triton X-(Sigma-Aldrich). After blocking with 5% BSA, fixed cells were incubated with rabbit anti-HIF-1α (1:100; Abcam, ab51608) and mouse anti-α-SMA (1:600; Abcam, ab7817) antibodies, followed by donkey anti-rabbit IgG Alexa Fluor 750-conjugated (1:1000; Abcam, ab175728) or goat anti-mouse IgG Alexa Fluor 555-conjugated (1:1000; Invitrogen, A21424) antibodies. Subsequently, cells were mounted in ProLong^{™} Diamond Antifade Mountant with DAPI (Invitrogen, P36971). Fluorescence images were taken under a Keyence BZ-800 Microscope (Keyence).

### 1-16. Immunohistochemical staining

Clinical tumor samples and mouse xenograft tumors were fixed in neutral formalin and embedded in paraffin. Immunohistochemical staining was performed as previously described. Briefly, sections were used for H&E staining or immunostaining with primary anti-FAPα (ab53066, Abcam, 1:100), anti-HIF-1α (ab51608, Abcam, 1:100) or anti-α-SMA (ab7817, Abcam, 1:500) antibodies, followed by staining with biotinylated secondary antibodies (Vector Laboratories) and incubation with the VECTASTAIN^{™} ABC kit (PK6100, Vector Laboratories) and 3,3'-diaminobenzidine (D12384, Sigma). For evaluation of HIF-1α staining, positive cells in four representative areas were counted using Hybrid Cell Count software (Keyence).

### 1-17. Processing of snRNA-seq data

The gene count matrices were analyzed by Seurat software v3.2.2 running on R v3.6.0. The following cells were removed from the dataset: cells with more than 1% mitochondrial gene counts; cells with more than 6,000 unique feature counts; and cells with less than 400 unique feature counts. The gene-barcode matrix of the filtered cells was normalized using "LogNormalize." The top 2,000 variable genes were then identified using the "vst" method in the Seurat FindVariableFeatures function. All cells from ten OCCC samples were integrated using the FindIntegrationAnchors and IntegrateData functions in Seurat. After filtering of cells and data integration, a total of 62,673 cells were subjected to scaling using the Seurat ScaleData function. Subsequently, the scaled data were analyzed by PCA using the Seurat RunPCA function, with npcs = 30 parameters. UMAP plots were generated by the Seurat RunUMAP function, with dims = 1:30.

### 1-18. Annotation of cell populations in OCCC

To stratify cell populations using the integrated snRNA-seq data, low-resolution clustering was performed using the FindClusters function, with a resolution of 0.2. To annotate the five classified cell populations, specific marker genes were used to identify cell populations corresponding to epithelial cell and non-tumor cell populations. Annotation of these cell populations was confirmed by examining expression of various marker genes.

### 1-19. Copy number inference from sequencing data

InferCNV (https://github.com/broadinstitute/inferCNV) was used to analyze large-scale chromosome copy number alterations based on the single-cell sequencing data. The InferCNV pattern in each chromosome was examined in epithelial cells, using non-tumor cells (CAFs and endothelial cells) as a reference.

### 1-20. Enrichment analysis

To perform ssGSEA of cancer subpopulations, measurement of the signature scores for hallmark gene sets expressed by each cancer subpopulation was based upon ssGSEA of the single-nucleus RNA-seq data. ssGSEA was performed using escape (v1.8.0, http://www.bioconductor.org/packages/release/bioc/vignettes/escap e/inst/doc/vignette.html) running on R v4.2.1. To perform GO enrichment analyses, differentially expressed genes (DEGs) were selected using the FindAllMarkers function in Seurat. Subsequently, clusterProfiler (v4.2.2) used the DEGs in each subpopulation to identify the top 10 most significant GO terms in biological processes (BP) categories.

### 1-21. Quantification of transcription factor activity

The activity of major transcription factors in each cell was inferred by VIPER (Virtual Inference of Protein-activity by Enriched Regulon analysis) v1.30.0 running on R v4.2.1. Transcription factor-target interactions classified as confidence level A (DoRothEA v1.6.0) were used to calculate VIPER scores. VIPER scores were visualized in violin plots and heatmaps. Transcription factor-target interactions of the Cancer #2 cluster were depicted by the igraph package in R.

### 1-22. Prognosis analysis

The top 20 DEGs that were selected using the FindAllMarkers function in Seurat were defined as signature genes for each cancer subpopulation. Surgical specimens of thirty advanced stage OCCC (Stages II-IV) were subjected to bulk RNA-seq analyses, and the patients were classified into two groups based upon average expression of the signature genes. Kaplan-Meier analysis was performed using the "Survival" package in R to evaluate the prognostic value of cancer cell clusters. p values for overall survival and progression-free survival were evaluated using a stratified log rank test.

### 1-23. Ligand-receptor interaction analysis

Ligand-receptor interaction analysis based on snRNA-seq data was performed by NicheNet. Ligands and receptors were selected from the DEGs of each cell population using the FindAllMarkers function in Seurat. The selected ligands from the Cancer #2 subpopulation were used to identify paired receptors from the DEGs of non-tumor cells based on the NicheNet ligand-receptor network. The Seurat AverageExpression function was used to visualize average expression of the ligands and receptors of each population in the heatmap. Subsequently, the interaction potential between the selected ligand-receptor pairs was estimated using NicheNet weighted integrated networks.

### 1-24. Spatial transcriptomics data processing

The Visium spot-gene expression matrices and spatial information of spatial transcriptomics data were imported into Seurat v3.2.0 for downstream analysis. The SCTransform function in Seurat was used to normalize the UMI count in each spot. The object was run through PCA using the Seurat RunPCA function, with npcs = 20 parameters, and UMAP plots were generated by the Seurat RunUMAP function, with dims = 1:20. Clustering of the Visium spots was performed using the FindClusters function with resolution = 20.

### 1-25. Integration of snRNA-seq and spatial transcriptomics data

An anchor-based integration method in Seurat v3.2.0 was used to integrate snRNA-seq and Visium data. Transfer anchors were detected using the Seurat FindTransferAnchors function, setting the combined snRNA-seq datasets as a reference and one of the Visium datasets as a query. Following integration, the cluster labels of snRNA-seq datasets were transferred to the spatial dataset using the Seurat TransferData function, thereby providing a prediction score for each snRNA-seq cluster per spot.

### 1-26. Processing of scRNA-seq data

The gene count matrices were imported to Seurat software v3.2.2 running on R v3.6.0. The following cells were removed from the dataset: cells with more than 10% mitochondrial gene counts; cells with more than 6,000 unique feature counts; and cells with less than 200 unique feature counts. The filtered gene-barcode matrix was normalized using "LogNormalize" in Seurat. Subsequently, the top 2,000 variable genes were identified using the "vst" method in the Seurat FindVariableFeatures function. Data obtained from co-cultured and monocultured cells were merged using the Seurat merge function. After filtering and merging, a total of 8,208 cells were processed for the following analysis: The merged object was scaled via the Seurat ScaleData function and run through PCA using the Seurat RunPCA function. UMAP plots were generated by the Seurat RunUMAP function, with dims = 1:30.

### 1-27. Image analysis of multiplexed immunofluorescence

QuPath (version 0.2.1) was used to measure immunofluorescence intensity in tumor and non-tumor cells. After loading whole images, cells were segmented using StarDist, and the fluorescence intensity of each cell was measured. Subsequently, cancer cells and CAFs were identified based on expression of PAX8 and α-SMA, respectively. An identical threshold intensity for immunofluorescence signals was applied for all samples. The centroid distances between α-SMA (+) cells and PAX8 (+)/HIF-1α (+) cells were estimated using the "Detect centroid distance 2D" command. After annotation of each cell, data were exported into CytoMAP (version 1.4.21).

### 2. Results

### 2-1. Identification of a cancer cell subpopulation associated with chemoresistance of OCCC

To identify intratumor networks responsible for chemoresistance of OCCC, frozen samples were obtained from surgical specimens, and integrative analyses that combine single-cell analyses and spatial transcriptomics were performed. Subsequently, the analyses were extended by multicolor quantitative immunostaining, *in vitro* co-cultures, and mouse xenograft experiments.

To obtain single-cell transcriptome data from frozen samples, chemosensitive (n = 5, OCC-S1-5) and chemoresistant (n = 5, OCC-R1-5) cases were analyzed. The snRNA-seq data were then subjected to dimensionality reduction using uniform manifold approximation and projection (UMAP). Clustering of single-nucleus data presented on UMAP indicated that cells were stratified mainly according to clinical case, which was presumably caused by batch effects. To eliminate batch effects and integrate individual datasets, an anchoring procedure was performed so that cell identities could be compared across samples.

After anchoring the data, it was found that major cell types (epithelial cancer cells, CAFs, endothelial cells, and immune cells) formed distinct clusters upon UMAP presentation. Cells from both chemoresistant and chemosensitive cases were distributed within each cell type. In the EpCAM⁺ epithelial cell population, non-tumor cells were barely detectable based on an estimation of copy number alterations by InferCNV analyses. This was presumably attributed to careful elimination of non-tumor tissues during sample preparation.

To examine potential links between chemoresistance and oncogenic activations, genomic alterations in major oncogenes and tumor suppressor genes were evaluated using the NCC Oncopanel. As reported previously, mutations of ARID1A and PIK3CA were identified in a large proportion of the samples (7 of 10 cases and 4 of 10 cases, respectively). However, there was no clear association with chemoresistance because both of these mutations occurred in both chemosensitive and chemoresistant cases.

Next, to examine whether a chemoresistant cancer subpopulation exists, the EpCAM⁺ tumor population was stratified into six subpopulations (Cancer #1-6). Remarkably, it was found that a fraction of the Cancer #2 subpopulation was higher in chemoresistant cases than in chemosensitive ones. On the other hand, evaluation of non-tumor cell types revealed no significant difference in numbers between chemoresistant and chemosensitive cases.

### 2-2. The chemoresistant population of OCCC is associated with HIF activation and a poor prognosis

To determine the gene expression profiles of each cancer subpopulation, preferentially expressed signature genes were isolated. Examination of signature gene expression in advanced OCCC cases (n = 30) indicated that the signature of the Cancer #2 subpopulation, but not that of other subpopulations, was associated with shortened progression-free or overall survival, linking the chemoresistance-associated Cancer #2 subpopulation to a poor prognosis.

Next, Gene Ontology (GO) enrichment analyses of the subpopulations were performed to investigate their biological characteristics. The results showed that the Cancer #2 subpopulation was associated with hypoxic responses and with the extracellular matrix. In accordance, single-sample gene set enrichment analyses (ssGSEA) of hallmark signature gene sets revealed that hypoxia pathways were activated specifically in the Cancer #2 subpopulation. On the other hand, enrichment analyses of the other major subpopulations indicated that the Cancer #1 and #3 subpopulations were associated with interferon response pathways and cell-cycle-related pathways, respectively, suggesting that the Cancer #3 subpopulation is a cycling population.

Next, virtual inference of protein activity by enriched regulon analysis (VIPER) was performed to investigate transcriptional regulators associated with each cluster. In agreement with the findings of an elevated hypoxic response, the Cancer #2 subpopulation showed elevated activity of HIF1A (HIF-1α) and EPAS1 (HIF-2α). Of note, the top five transcription factors activated in the Cancer #2 subpopulation (HIF1A, EGR-1, ATF-2, EPAS1(HIF-2A), and SP-1) mediate hypoxic responses. This suggests that these transcription factors co-operate to induce hypoxic responses. Taken together, these results indicate that the #2 chemoresistant subpopulation is associated with a poor prognosis and HIF-mediated hypoxic responses.

### 2-3. Chemoresistant cells are localized in CAF-populated areas of OCCC

Next, an attempt was made to determine the histological localization of the Cancer #2 chemoresistant cancer subpopulation by performing spatial transcriptomics analysis. Surgical specimens from chemoresistant (OCC-R2) and chemosensitive (OCC-S3) cases were subjected to Visium spatial gene expression analyses. Specific markers for epithelial cancer cells, CAFs, endothelial cells, and immune cells were used to determine the location of these cells within tumors. Hematoxylin and Eosin (H&E) staining of serial sections indicated that the specimens were roughly segregated into cancer- or CAF-dominated regions. Consistent with this, the tissue distribution of cancer cells and CAFs depicted by Visium analyses corresponded approximately to the cancer cell- or CAF-dominated regions visualized by H&E staining. Indeed, Visium spots could be classified into three groups based upon gene expression profiles: cancer-dominated, CAF-dominated, and cancer/CAF-mixed. The distribution of the three types of spots corresponded approximately to that of cancer cells and CAFs observed in the H&E images.

Next, to localize the major cancer subpopulations (#1-5), anchor-based integration of snRNA-seq data and Visium data was performed, and then a prediction score for each subpopulation in each Visium spot was calculated. In both chemoresistant and chemosensitive cases, visualization of cancer subpopulations based upon the prediction scores revealed that the Cancer #2 subpopulation was localized mainly in cancer/CAF-mixed spots. By contrast, the Cancer #1 and #3 populations were localized mainly in cancer-dominated spots.

In agreement with the snRNA-seq data, the Cancer #2 signature, but not the other signatures, was expressed to a greater extent in OCC-R2 than in OCC-S3, thereby supporting an association between cancer cells harboring the Cancer #2 signature and chemoresistance.

### 2-4. HIF-1α-induced cancer cells reside near CAFs in chemoresistant OCCC

Next, HIF-1α-positive cancer cells were immunostained to further investigate the location of the Cancer #2 chemoresistant populations in cancer/CAF-mixed areas. Remarkably, co-immunostaining of chemoresistant tumors (OCC-R1-5) with antibodies specific for HIF-1α, PAX8 (a marker for ovarian cancer cells), and α-SMA (a marker for CAFs) revealed widespread distribution of PAX8-positive cancer cells that co-express HIF-1α. By contrast, the fraction of cancer cells co-expressing detectable HIF-1α in chemosensitive tumors (OCC-S1-5) was much lower than in chemoresistant tumors. In fact, quantification of stained cells using QuPath indicated that the fraction of the HIF-1α-positive population in chemoresistant tumors was, on average, three times higher than that in the chemosensitive tumors (33.2% vs. 11.0%, respectively)

It was observed that HIF-1α-positive cancer cells in chemoresistant tumors frequently localized near α-SMA-positive cells. Indeed, evaluation of relative distance among HIF-1α-positive cancer cells, HIF-1α-negative cancer cells, and α-SMA-positive cells by nearest-neighbor analyses (CytoMAP) indicated the localization of HIF-1α-positive cancer cells near α-SMA-positive cells. These data suggest that localization of HIF-1α-induced cancer cells near CAFs is a hallmark of chemoresistant OCCC.

### 2-5. CAFs in chemoresistant tumors show a myofibroblastic phenotype

Close localization of CAFs to chemoresistant cells suggests that CAFs may play a functional role in enhancing the chemoresistance of OCCC. To examine a unique subpopulation of CAFs that may exist in chemoresistant OCCC, the snRNA-seq data were used to stratify the CAF population mentioned in 2-1 above. Contrary to stratification of the cancer population, however, a subpopulation that exists preferentially in chemoresistant OCCC was not found.

As an alternative approach, the snRNA-seq data were used to examine whether CAFs from chemosensitive cancers were associated with any particular biological features. It has been reported that CAFs comprise heterogeneous populations, including inflammatory CAFs (iCAFs), antigen-presenting CAFs (apCAFs), and myofibroblastic CAFs (myCAFs), and that myCAFs reside close to cancer cells. Comparison of CAFs from chemoresistant and chemosensitive tumors by ssGSEA and GO-term analyses indicated that CAFs from chemoresistant OCCC were associated with epithelial-mesenchymal transition (EMT) and extracellular matrix organization, phenotypes associated with myCAFs. Strikingly, CAFs from chemoresistant OCCC showed elevated expression of the myCAF gene signature and the myCAF-related genes FAP, TPM1, and THBS2. Furthermore, immunostaining studies revealed higher levels of FAP-α (a protein encoded by the FAP gene) in chemoresistant tumors than in chemosensitive ones. Collectively, these data indicate that the myCAF population is increased in chemoresistant cancer and that HIF-1α-induced cancer cells and myCAFs constitute a cancer microenvironment specific to chemoresistance.

### 2-6. In vitro co-cultivation of CAFs with chemoresistant OCCC cells recapitulates the chemoresistant niche

Co-localization of the chemoresistant subpopulation of cancer cells and CAFs implies mutual crosstalk between these cells in chemoresistant niches. To examine potential crosstalk between these cells, an *in vitro* co-culture system was established. First, cancer spheroids and CAFs were established separately from fresh surgical OCCC specimens. Second, chemoresistant cancer-derived spheroids were retrospectively selected based upon extensive expression of HIF-1α in cancer cells in the original surgical specimens. The identities of the selected cancer spheroids and CAFs were confirmed by expression of specific markers: PAX8 and KRT7 for OCCC and α-SMA and collagen I for CAF. Subsequently, the spheroids and CAFs were labeled with GFP and mCherry, respectively, and then cultivated alone or together (at a 1:1 ratio) to examine cell proliferation and phenotypic alterations induced by co-cultivation (Figs. 1A and 1B). Indeed, co-cultivation increased the survival rate of CAFs (Fig. 1C) and induced expression of HIF-1α and HIF-2α in cancer spheroid cells (Figs. 1D and 1E). Remarkably, co-culture increased chemoresistance to carboplatin (Fig. 1F), indicating that the presence of CAFs contributes to cancer chemoresistance.

Next, to examine changes in gene expression induced after co-culture, single-cell RNA-seq (scRNA-seq) of cells cultivated under monoculture or co-culture conditions was performed (Fig. 1G). When the gene expression profiles of cancer spheroids under co-culture and monoculture conditions were compared by ssGSEA, it was found that four of five top hallmark signatures (EMT, TNF-α signaling via NF-κB, inflammatory response, and hypoxia) induced after co-culture were identical to those upregulated in the Cancer #2 subpopulation. In addition, co-culture with CAFs caused specific upregulation of the Cancer #2 gene signature (Fig. 1H) and induced activation of all nine top transcription factors activated in the Cancer #2 subpopulation. Collectively, phenotypic alteration of cancer spheroids induced by co-cultivation with CAFs largely simulated the unique characteristics of the Cancer #2 subpopulation.

Gene expression profiles of CAFs under co-culture and monoculture conditions were also compared by ssGSEA. The EMT pathway, which was strongly upregulated in CAFs in chemoresistant OCCC, was induced under co-culture conditions. In addition, it was found that TGF-β signaling was strongly induced after co-cultivation. This suggests that TGF-β signaling, which is a well-known signaling pathway that drives CAF generation, may account for the EMT phenotype of the CAFs induced under co-culture conditions. Furthermore, co-cultivation upregulated the myCAF signature (Fig. 1I) as well as genes representative of myCAF, including FAP, THBS2, and TPM1, induction of which was also observed in chemoresistant OCCC. Thus, both cancer spheroids and CAFs, when incubated together, undergo phenotypic alterations associated with chemoresistant cancer. These data strongly suggest that interactions between cancer cells and CAFs lead to formation of a chemoresistant niche in cancer.

### 2-7. CAF activation by cancer-derived PDGF mediates chemoresistance and activates HIF-1α in cancer cells

To better understand the molecular mechanism underlying chemoresistance mediated by the cancer-CAF interaction, NicheNet was used to examine potential ligand-receptor interactions between these cells. Examination of the snRNA-seq data identified 12 ligand-coding genes that were highly expressed in the Cancer #2 subpopulation (NAMPT, EFNA5, PDGFB, C3, ANXA1, SPP1, FN1, ITGB1, LAMC2, LAMB1, LAMA1, and RELN). Next, whether the gene encoding the receptor was highly expressed by CAFs was examined. Receptor-ligand analyses revealed that interaction between PDGFB (the β-subunit of PDGF) and PDGFRB (the β-subunit of the PDGF receptor) was a strong candidate mediator of cell-cell signaling between cancer cells and CAFs.

Therefore, the functional significance of the PDGF-PDGFR interaction in the co-culture system was next examined. As expected, PDGFB and PDGFRB were expressed at high levels by cancer spheroid cells and CAFs, respectively (Fig. 2A). Co-culture led to activating phosphorylation of PDGFR (p-PDGFRB) and expression of a myCAF marker (FAP-α) in CAFs (Fig. 2B). Indeed, treatment of CAFs with a purified PDGFB ligand induced phosphorylation of PDGFRB and expression of FAP-α (Fig. 2C) and increased proliferation of CAFs (Fig. 2D). Conversely, CRISPR-mediated knockout of PDGFRB in CAFs abolished PDGF-mediated proliferation (Figs. 2E and 2F) and FAP-α expression (Fig. 2G). This indicates that the PDGFB-induced proliferation and expression of the myCAF-like phenotype were mediated by activation of the PDGF receptor in CAFs.

Notably, immunostaining of chemoresistant OCCC showed activating phosphorylation of PDGFRB in α-SMA-positive CAFs that resided near KRT7-positive cancer cells. This suggests that PDGFR signaling in CAFs is activated by adjacent cancer cells *in vivo*.

Next, the functional role of PDGFR signaling in cancer chemoresistance was examined using a co-culture system. PDGFR knockout in CAFs reduced its viability (Fig. 2H). Surprisingly, the PDGFRB knockout in CAFs inhibited expression of HIF-1α, HIF-2α, and PDGFB in cancer cells (Fig. 2I). Moreover, the knockout reduced cancer chemoresistance to carboplatin (Fig. 2J). Taken together, these data indicate the presence of a positive feedback loop between cancer cells and CAFs: PDGF expressed by OCCC induces PDGFR-mediated activation and survival of CAFs, which, in turn, augments HIF activation, PDGF expression, and chemoresistance in cancer cells.

### 2-8. CAF inhibition by ripretinib in combination with carboplatin inhibits cancer growth

Because of the crucial role of the PDGF-PDGFR signaling axis in CAF-mediated chemoresistance, a new therapy targeting PDGFR signaling in CAFs was devised. Carboplatin did not have any significant effect on CAF growth (Figs. 3A, 4A, and 4B). On the other hand, TKIs, which can inhibit PDGFR, showed varying levels of growth inhibition (Figs. 3A, 4A, and 4B). 1 µM ripretinib inhibited activating phosphorylation of PDGFRB and expression of FAP-α within 24 hours. Subsequently, the inhibitory effects of ripretinib in the presence or absence of carboplatin were examined in the *in vitro* co-culture system. As expected, ripretinib at 1-5 µM effectively reduced the viability of CAFs even in the absence of carboplatin (Fig. 3B). Importantly, inhibition of cancer cell proliferation by carboplatin was markedly enhanced by ripretinib (Figs. 3C and 3D). By contrast, ripretinib did not show significant enhancement of carboplatin-mediated inhibition upon monoculture of cancer cells (Fig. 3E). This indicates that ripretinib inhibits cancer growth via CAF suppression.

Finally, the effect of combined treatment with carboplatin and ripretinib was examined. The cancer spheroids and CAFs used in the co-culture assays were mixed at a 1:1 ratio and transplanted subcutaneously into immunocompromised NOG mice. Notably, ripretinib in combination with carboplatin led to marked inhibition of tumor growth (Fig. 3F).

Thus, ripretinib inhibits growth of chemoresistant cancer by inhibiting CAFs. Therefore, it was predicted that a fraction of HIF-1α-positive cancer cells would be reduced in the presence of ripretinib. Indeed, a marked reduction was observed in a fraction of HIF-1α-positive cancer cells (Figs. 3G and 3H) as well as α-SMA-positive CAFs after treatment. These data indicate that CAF inhibition is an effective treatment that eliminates chemoresistant cancer when combined with standard chemotherapy agents.

## Claims

1. A cancer-associated fibroblast inhibitor comprising a PDGFR inhibitor.

2. The cancer-associated fibroblast inhibitor according to claim 1, wherein the PDGFR inhibitor is at least one selected from the group consisting of a PDGFR function inhibitor and a PDGFR expression inhibitor.

3. The cancer-associated fibroblast inhibitor according to claim 1, wherein the PDGFR inhibitor is at least one selected from the group consisting of a low-molecular-weight compound, a polynucleotide targeting PDGFR, an expression cassette for the polynucleotide, a peptide, a protein, and an antibody.

4. The cancer-associated fibroblast inhibitor according to claim 1, wherein the PDGFR inhibitor is a low-molecular-weight compound, and the low-molecular-weight compound is a kinase inhibitor.

5. The cancer-associated fibroblast inhibitor according to claim 4, wherein the kinase inhibitor is at least one selected from the group consisting of ripretinib, ponatinib, erdafitinib, dovitinib, lenvatinib, foretinib, ENMD-2076, PP121, and cediranib.

6. The cancer-associated fibroblast inhibitor according to any one of claims 1 to 5, wherein the cancer-associated fibroblasts are cells in ovarian cancer tissue, breast cancer tissue, or colon cancer tissue.

7. The cancer-associated fibroblast inhibitor according to any one of claims 1 to 5, wherein the cancer-associated fibroblasts are cells in ovarian cancer tissue.

8. The cancer-associated fibroblast inhibitor according to any one of claims 1 to 5, for use in combined administration with an anticancer agent.

9. The cancer-associated fibroblast inhibitor according to claim 8, wherein the anticancer agent is a platinum-based drug.

10. An enhancer of the anticancer effect of an anticancer agent, the enhancer comprising a PDGFR inhibitor.

11. A preventive or therapeutic agent for at least one cancer selected from the group consisting of ovarian cancer, breast cancer, colon cancer, pancreatic cancer, urothelial cancer, prostate cancer, esophageal cancer, liver cancer, kidney cancer, uterine cancer, stomach cancer, glioblastoma, lung cancer, and melanoma, the preventive or therapeutic agent comprising a PDGFR inhibitor.

12. The preventive or therapeutic agent according to claim 11, for use in combined administration with an anticancer agent.
